(19) **Europäisches Patentamt / European Patent Office / Office européen des brevets**

(11) **EP 3 219 674 B1**

(12) **EUROPEAN PATENT SPECIFICATION**

(45) Date of publication and mention
of the grant of the patent:
**25.09.2019 Bulletin 2019/39**

(21) Application number: **15858877.2**

(22) Date of filing: **11.11.2015**

(51) Int Cl.:
*A61K 8/25* (2006.01)        *A61K 8/02* (2006.01)
*C09C 1/04* (2006.01)        *A61K 8/27* (2006.01)
*A61Q 1/04* (2006.01)        *A61Q 1/08* (2006.01)
*A61Q 1/10* (2006.01)        *A61Q 17/04* (2006.01)
*A61Q 19/00* (2006.01)       *C01G 9/02* (2006.01)
*A61Q 1/02* (2006.01)

(86) International application number:
**PCT/JP2015/081720**

(87) International publication number:
**WO 2016/076350 (19.05.2016 Gazette 2016/20)**

(54) **SILICON OXIDE-COATED ZINC OXIDE AND METHOD FOR PREPARING SAME, AND COMPOSITION AND COSMETIC MATERIAL CONTAINING SILICON OXIDE-COATED ZINC OXIDE**

SILICIUMOXIDBESCHICHTETES ZINKOXID UND VERFAHREN ZUR HERSTELLUNG DAVON UND ZUSAMMENSETZUNG UND KOSMETISCHES MATERIAL MIT SILICIUMOXIDBESCHICHTETEM ZINKOXID

OXYDE DE ZINC ENDUIT D'OXYDE DE SILICIUM ET PROCÉDÉ POUR LE PRÉPARER, COMPOSITION CONTENANT DE L'OXYDE DE ZINC ENDUIT D'OXYDE DE SILICIUM, ET MATÉRIAU COSMÉTIQUE CONTENANT DE L'OXYDE DE ZINC ENDUIT D'OXYDE DE SILICIUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **13.11.2014   JP 2014230654**
      **15.07.2015   JP 2015141392**

(43) Date of publication of application:
**20.09.2017 Bulletin 2017/38**

(73) Proprietor: **Sumitomo Osaka Cement Co., Ltd.**
**Tokyo 102-8465 (JP)**

(72) Inventors:
• **SUMA, Syunsuke**
  **Tokyo 102-8465 (JP)**
• **ITAGAKI, Tetsuro**
  **Tokyo 102-8465 (JP)**

• **MATSUSHITA, Hirokazu**
  **Tokyo 102-8465 (JP)**

(74) Representative: **Hoffmann Eitle**
**Patent- und Rechtsanwälte PartmbB**
**Arabellastraße 30**
**81925 München (DE)**

(56) References cited:
EP-A1- 0 824 086      WO-A1-2014/171322
JP-A- H0 789 710      JP-A- H08 104 512
JP-A- H10 130 021     JP-A- H11 302 015

• **H. WANG ET AL.: 'Effect of Polyelectrolyte Dispersants on the Preparation of Silica-Coated Zinc Oxide Particles in Aqueous Media' J. AM. CERAM. SOC. vol. 85, no. 8, 01 August 2002, pages 1937 - 1940, XP009143705 DOI: 10.1111/J.1151-2916.2002.TB00384.X**

## Description

Technical Field

The present invention relates to silicon oxide-coated zinc oxide and a method for preparing the same, a composition containing silicon oxide-coated zinc oxide, and a cosmetic material.

Background Art

Since zinc oxide has photocatalytic activity, it is necessary to carefully select the amount blended or other coexisting components being blended when zinc oxide is used for cosmetic materials. Therefore, for the purpose of suppressing the photocatalytic activity of zinc oxide, surface-coated zinc oxide obtained by coating the surface of zinc oxide with a poorly active substance such as silicon oxide or aluminum oxide is proposed (for example, refer to Patent Literature Nos. 1 and 2) .

In recent years, compared with oil-based cosmetic materials, water-based cosmetic materials are not sticky and are capable of obtaining a fresh feeling, and thus water-based cosmetic materials have been frequently used as a variety of cosmetic materials such as sunscreens, emulsions, and creams.

However, in a case in which zinc oxide is used in water-based cosmetic materials, problems unique to water-based cosmetic materials are caused.

The problems are caused because zinc oxide is an ampholytic metal oxide. Examples of the problems include a problem of the elution of zinc ions caused by the easy dissolution of zinc oxide in acids or alkalis and a problem of the elution of zinc ions caused by the dissolution of a small amount of zinc oxide in water. Therefore, in a case in which zinc oxide is used for water-based cosmetic materials, zinc ions being eluted react with organic ultraviolet absorbers, water-soluble polymers such as viscosity improvers, and the like, and there is a concern that problems of the degradation of performance, discoloration, changes in viscosity, and the like as cosmetic materials are caused. As a result, there has been a problem in that the degree of freedom in formulation is limited in water-based cosmetic materials.

Particularly, when a carbomer such as carboxy vinyl polymer which is generally used as a viscosity improver and zinc oxide are jointly used, zinc ions being eluted and carboxylate groups ($COO^-$) of the carbomer react with each other, and thus the gel structure of the carbomer breaks, and there is a problem in that the viscosity decreases.

Therefore, in order to suppress the elution of zinc ions, a surface-coated zinc oxide obtained by coating the surface with an inorganic compound such as silicon oxide is proposed (for example, refer to Patent Literature Nos. 3 and 4).

However, even in the methods described in Patent Literature Nos. 3 and 4, there is a problem in that it is difficult to sufficiently suppress the elution of zinc ions from zinc oxide.

Therefore, in Patent Literature No. 5, a method in which, when the abundance ratio of silicon in a silicon oxide coating in a $Q^3$ environment is indicated by $Q^3$, and the abundance ratio in a $Q^4$ environment is indicated by $Q^4$, $Q^3+Q^4 \geq 0.6$ and $Q^4/(Q^3+Q^4) \geq 0.5$ are set, and the elution amount of zinc ions is decreased is proposed.

EP 0 824 086 A1 relates to a stabilised aqueous dispersion of particulate zinc oxide comprising water, a stabilising agent and zinc oxide, the particles of which are coated with dense amorphous silica. Useful stabilising agents are said to include cellulose ethers which contain quaternary ammonium groups and polyalkylene glycols.

Citation List

Patent Literature

[Patent Literature No. 1] Japanese Patent No. 2851885
[Patent Literature No. 2] Japanese Patent No. 3848458
[Patent Literature No. 3] Japanese Patent No. 3520785
[Patent Literature No. 4] Japanese Patent No. 4836232
[Patent Literature No. 5] Pamphlet of International Publication No. WO2014/171322

Summary of Invention

Technical Problem

However, in Patent Literature No. 5, a silica coating is formed using alkoxysilane or a silicate alkali metal salt and alkoxysilane, but it is difficult to decrease the elution amount of zinc ions using a silicate alkali metal salt alone.

The present invention has been made in consideration of the above-described circumstances, and an object of the present invention is to provide silicon oxide-coated zinc oxide in which, even in a silica coating formed of a silicate alkali metal salt, the elution of zinc ions is suppressed, a method for preparing the same, a composition containing silicon oxide-coated zinc oxide, and a cosmetic material.

Solution to Problem

The present inventors repeated intensive studies in order to achieve the above-described objects and consequently found the following invention. That is, it was found that, when the average primary particle diameter of zinc oxide particles is set to more than 50 nm and 500 nm or less, it is possible to suppress the elution of zinc ions even in silica coatings formed of a silicate alkali metal salt.

That is, silicon oxide-coated zinc oxide of the present invention is silicon oxide-coated zinc oxide as presently claimed formed by coating surfaces of zinc oxide particles with a silicon oxide coating, wherein an average primary particle diameter of the zinc oxide particles is more than 50 nm and 500 nm or less, and, when held in a buffer solution having a pH of 5 for 1 hour, an elution ratio S of the zinc oxide defined by following Expression (1) is 50% or less.

$$\text{Elution ratio S} = (\text{amount of eluted zinc oxide})/(\text{amount of zinc oxide in silicon oxide-coated zinc oxide}) \cdots \quad (1)$$

A method for preparing silicon oxide-coated zinc oxide of the present invention, as presently claimed comprises: mixing a zinc oxide water-based suspension including zinc oxide particles having an average primary particle diameter of more than 50 nm and 500 nm or less with a silicate alkali metal salt aqueous solution including a silicate alkali metal salt so as to prepare a water-based suspension including the zinc oxide particles and the silicate alkali metal salt, and adding an acid to the water-based suspension so as to cause a reaction; and thermally treating the obtained reaction product at a temperature of 200°C or higher and lower than 600°C for one to five hours.

A composition containing silicon oxide-coated zinc oxide of the present invention comprises the silicon oxide-coated zinc oxide of the present invention.

A cosmetic material of the present invention comprises at least one of the silicon oxide-coated zinc oxide of the present invention and the composition containing silicon oxide-coated zinc oxide of the present invention in a base.

Advantageous Effects of Invention

According to the silicon oxide-coated zinc oxide of the present invention, since zinc oxide particles having an average primary particle diameter of more than 50 nm and 500 nm or less are used, it is possible to suppress the elution of zinc ions even in silica coatings formed of a silicate alkali metal salt.

According to the method for preparing silicon oxide-coated zinc oxide of the present invention, since silica coatings are formed using zinc oxide particles having an average primary particle diameter of more than 50 nm and 500 nm or less, it is possible to prepare silicon oxide-coated zinc oxide in which the elution of zinc ions is suppressed.

According to the composition containing silicon oxide-coated zinc oxide of the present invention, since the silicon oxide-coated zinc oxide of the present invention is included, in the case of being applied not only to oil-based cosmetic materials such as water-in-oil (W/O)-type materials but also to water-based cosmetic materials, it is possible to suppress the elution of zinc ions.

According to the cosmetic material of the present invention, since at least one of the silicon oxide-coated zinc oxide of the present invention and the composition containing silicon oxide-coated zinc oxide of the present invention is included in the base, in the case of being applied not only to oil-based cosmetic materials such as water-in-oil (W/O)-type materials but also to water-based materials, it is possible to suppress the elution of zinc ions.

Brief Description of Drawings

FIG. 1 is a view illustrating a change in the pH of silicon oxide-coated zinc oxide of Example 1 in a citric acid-containing aqueous solution.
FIG. 2 is a view illustrating changes in the viscosity of compositions of Example 6 and Comparative Examples 7 to 9.

Description of Embodiments

The present invention relates to silicon oxide-coated zinc oxide and a method for preparing the same, and a composition and a cosmetic material containing silicon oxide-coated zinc oxide. In more detail, the present invention relates to silicon oxide-coated zinc oxide that is preferably used for a variety of cosmetic products such as skincare cosmetic products, makeup cosmetic products, and bodycare cosmetic products, particularly, skin lotion, sunscreens, emulsions, cream, foundation, lipsticks, blushes, eyeshadow, and the like, a method for preparing the same, a composition containing silicon oxide-coated zinc oxide, and a cosmetic material including at least one of silicon oxide-coated zinc oxide and a composition containing silicon oxide-coated zinc oxide in a base.

Embodiments of a silicon oxide-coated zinc oxide and a method for preparing the same, and a composition and a cosmetic material containing silicon oxide-coated zinc oxide of the present invention will be described.

Meanwhile, the present embodiments are the specific descriptions for the better understanding of the gist of the present invention and do not limit the present invention unless particularly otherwise described.

[Silicon oxide-coated zinc oxide]

Silicon oxide-coated zinc oxide of the first embodiment of the present invention as claimed is silicon oxide-coated zinc oxide formed by coating surfaces of zinc oxide particles with a silicon oxide coating, wherein the amount of silicon oxide in the silicon oxide-coated zinc oxide is 12% by mass or more and wherein an average particle diameter of the zinc oxide particles is more than 50 nm and 500 nm or less, and, when held in a buffer solution having a pH of 5 for 1 hour, an elution ratio S of the zinc oxide defined by following Expression (1) is 50% or less.

$$\texttt{Elution ratio S=(amount of eluted zinc oxide)/(amount of}$$
$$\texttt{zinc oxide in silicon oxide-coated zinc oxide)} \cdots \quad (1)$$

When the silicon oxide-coated zinc oxide is held in a buffer solution having a pH of 5 for 1 hour, the elution ratio S of the zinc oxide defined by Expression (1) is 50% or less, preferably 40% or less, and more preferably 30% or less.

The elution of zinc oxide from the silicon oxide-coated zinc oxide occurs at pinholes being generated in a case in which the homogeneity of the silicon oxide coating is poor or thin portions in the coating. In a case in which the elution ratio S exceeds 50%, the homogeneity of silicon oxide in the silicon oxide-coated zinc oxide is poor, and the number of pinholes or thin portions in the coating increases. As a result, there is a concern that it may not be possible to sufficiently suppress the elution of zinc ions, which is not preferable.

A method for measuring the amount of zinc oxide eluted is not particularly limited, and it is possible to use, for example, an atomic absorption analysis, an ICP analysis, UV methods in which an indicator such as zincon or xylenol orange is used, and the like.

A method for measuring the amount of zinc oxide in the silicon oxide coating is not particularly limited, and it is possible to use an atomic absorption analysis, an ICP analysis, UV methods in which an indicator such as zincon or xylenol orange is used, and the like.

The average particle diameter of the silicon oxide-coated zinc oxide of the present embodiment is preferably more than 50 nm or 2,000 nm or less, more preferably 100 nm or more and 500 nm or less, and still more preferably 200 nm or more and 300 nm or less.

Here, the reasons for setting the average particle diameter of the silicon oxide-coated zinc oxide to the above-described range are as follows. At an average particle diameter of 50 nm or less, the average particle diameters of zinc oxide particles being included are also small, and it is not possible to suppress the elution of zinc ions. On the other hand, when the average particle diameter exceeds 2,000 nm, in a case in which the silicon oxide-coated zinc oxide is used for cosmetic materials and the like, there is a concern that squeaks and the like may be caused and a feeling during use may deteriorate.

As the average particle diameter of the silicon oxide-coated zinc oxide decrease, in the case of being blended into cosmetic materials, the silicon oxide-coated zinc oxide is suitable to enhance transparency during use. On the other hand, as the average particle diameter increases, the scattering intensity of ultraviolet rays also increases, and it is possible to shield ultraviolet rays having long wavelengths. Therefore, the average particle diameter of the silicon oxide-coated zinc oxide of the present embodiment may be appropriately selected in accordance with the intended transparency of cosmetic materials and the intended shielding properties of ultraviolet rays.

Meanwhile, the "average particle diameter of the silicon oxide-coated zinc oxide" in the present embodiment is a value obtained by the following method. That is, in a case in which the silicon oxide-coated zinc oxide of the present embodiment is observed using a transmission electron microscope (TEM) or the like, a predetermined number of the silicon oxide-coated zinc oxide particles, for example, 200 or 100 silicon oxide-coated zinc oxide particles are selected.

In addition, the longest straight line portions (maximum length diameters) of the respective silicon oxide-coated zinc oxide particles are measured, and these measurement values are weight-averaged.

Here, in a case in which the silicon oxide-coated zinc oxide particles agglomerate together, instead of measuring the agglomerated particle diameters of the agglomerates, the particle diameters of a predetermined number of particles (primary particles) of the silicon oxide-coated zinc oxide constituting the agglomerates are measured, and the average particle diameter is obtained.

The content rate of silicon oxide in the silicon oxide-coated zinc oxide of the present embodiment is 12% by mass or more and 50% by mass or less, more preferably 13% by mass or more and 40% by mass or less, and still more preferably 14% by mass or more and 35% by mass or less.

Here, when the content rate of silicon oxide in the silicon oxide-coated zinc oxide is more than 50% by mass, the content of zinc oxide particles in the silicon oxide-coated zinc oxide decreases. As a result, when attempts are made to obtain a desired ultraviolet shielding effect in cosmetic materials including the above-described silicon oxide-coated zinc oxide in a base, it is necessary to use a large amount of silicon oxide-coated zinc oxide, which is not preferable.

On the other hand, in a case in which the content rate of silicon oxide in the silicon oxide-coated zinc oxide is less than 12% by mass, it becomes difficult to uniformly coat the surfaces of zinc oxide particles with a silicon oxide coating. As a result, there is a concern that it may be impossible to sufficiently suppress the elution of zinc ions, which is not preferable.

When the content rate of silicon oxide is set in the above-described range, it is possible to suppress the elution of zinc ions and obtain silicon oxide-coated zinc oxide having excellent ultraviolet shielding performance.

The silicon oxide-coated zinc oxide of the present embodiment preferably includes at least one selected from the group consisting of Mg, Ca, and Ba.

The content rate of the alkali metal in the silicon oxide-coated zinc oxide of the present embodiment is preferably 0% by mass or more and 0.4% by mass or less, more preferably 0.01% by mass or more and 0.3% by mass or less, and still more preferably 0.01% by mass or more and 0.2% by mass or less.

The total content rate of at least one selected from the group consisting of Mg, Ca, and Ba in the silicon oxide-coated zinc oxide of the present embodiment is preferably 0.01% by mass or more and 1% by mass or less, more preferably 0.01% by mass or more and 0.5% by mass or less, and still more preferably 0.01% by mass or more and 0.3% by mass or less.

Here, the reasons for setting the content of the alkali metal in the silicon oxide-coated zinc oxide and the total content of at least one selected from the group consisting of Mg, Ca, and Ba in the silicon oxide-coated zinc oxide in the above-described ranges are as described below. A cause of changes in the hydrogen-ion exponent (pH) of the silicon oxide-coated zinc oxide of the present embodiment in the initial phase is not the elution of zinc ions, but, mainly, the elution of alkali metal ions being included in the silicon oxide-coated zinc oxide.

That is, in a case in which a silica coating is formed using a silicate alkali metal salt, silicate alkali metals and the like are included in the silicon oxide-coated zinc oxide (silicon oxide coating) . Therefore, in a case in which the silica coating is mixed with water-based materials, alkali metal ions are eluted into water, and the pH is changed.

Therefore, alkali metals being included in the silicon oxide coating are substituted with at least one selected from the group consisting of Mg, Ca, and Ba. As a result, the silicon oxide coating includes at least one selected from the group consisting of Mg, Ca, and Ba which are not easily freed instead of alkali metals which are easily freed in water. Therefore, it is possible to suppress the elution of components that change pH even when being mixed with water-based materials.

Meanwhile, the content of Na, Mg, Ca, and Ba in the silicon oxide-coated zinc oxide of the present embodiment can be measured by means of atomic absorption analyses.

In the silicon oxide-coated zinc oxide of the present embodiment, when the abundance ratio of silicon in the silicon oxide coating in a $Q^3$ environment is indicated by $Q^3$, and the abundance ratio in a $Q^4$ environment is indicated by $Q^4$, it is preferable that $Q^3+Q^4 \geq 0.6$ is satisfied and $Q^4/(Q^3+Q^4)<0.5$ is satisfied.

The values of $Q^3$ and $Q^4$ can be learned using the following method. That is, the NMR spectrum of the silicon oxide-coated zinc oxide is measured by means of MAS-nuclear magnetic resonance (NMR) spectroscopy in which solid $^{29}Si$ is used. In addition, the area ratios of signals attributed to individual environments of $Q^0$, $Q^1$, $Q^2$, $Q^3$, and $Q^4$ are measured from the peak area ratios of the NMR spectrum, whereby the values of $Q^3$ and $Q^4$ can be easily calculated.

In the silicon oxide-coated zinc oxide of the present embodiment, the amount of acetone being generated by the following evaluation method is preferably 30 ppm or less, more preferably 25 ppm or less, and still more preferably 20 ppm or less.

Since zinc oxide particles have photocatalytic activity, when ultraviolet rays are radiated to a 2-propanol aqueous solution including zinc oxide particles, the zinc oxide particles oxidize 2-propanol, thereby generating acetone. Therefore, the fact that the amount of acetone being generated by the following evaluation method is 30 ppm or less means that the photocatalytic activity of the zinc oxide particles is suppressed. That is, silicon oxide-coated zinc oxide from which the above-described result can be obtained can be said to have surfaces of zinc oxide particles that are coated with a homogeneous silicon oxide coating enough to preferably suppress the photocatalytic activity of the zinc oxide particles. As a result, it is considered that homogeneously-formed silicon oxide coatings are capable of suppressing the outward elution of zinc ions from the silicon oxide-coated zinc oxide. Therefore, the amount of acetone being generated by the following evaluation method can be used as an indicator of the elution suppression of zinc ions in the silicon oxide-coated zinc oxide.

(Evaluation method)

0.01 g of the silicon oxide-coated zinc oxide of the present embodiment is mixed into 9.99 g of an aqueous solution containing 300 ppm of 2-propanoland is ultrasonic-dispersed, thereby producing a suspension.

Next, 0.3 g of the ultrasonic-dispersed suspension and 2.7 g of the aqueous solution containing 300 ppm of 2-propanol were fed into a 10 mm×10 mm silica cell, thereby producing 3 g of a sample liquid. The sample liquid is irradiated using an ultraviolet lamp (central wavelength: 254 nm) at an irradiation distance of 10 cm for 6 hours, and then a supernatant liquid is collected.

Next, the amount of acetone being included in the collected supernatant liquid is determined using gas chromatography.

[Zinc oxide]

In the silicon oxide-coated zinc oxide of the present embodiment, the average primary particle diameter of the zinc oxide particles is more than 50 nm and 500 nm or less, preferably 100 nm or more and 400 nm or less, and more preferably 200 nm or more and 300 nm or less.

In addition, the primary particle diameters of 90% or more of zinc oxide particles out of these zinc oxide particles is preferably 100 nm or more and 400 nm or less, more preferably 200 nm or more and 400 nm or less, and still more preferably 200 nm or more and 300 nm or less.

When the average primary particle diameters of the zinc oxide particles are set in the above-described range, it is possible to suppress the elution of zinc ions from the silicon oxide-coated zinc oxide, and, in a case in which the silicon oxide-coated zinc oxide is applied to cosmetic materials, it is possible to improve a feeling during use while maintaining transparency.

In addition, when the average primary particle diameters of 90% or more, more preferably 95% or more, and still more preferably 97% or more of the zinc oxide particles are set in the above-described range, effects as described below can be obtained. That is, an effect of shielding ultraviolet rays, particularly, long-wavelength ultraviolet rays (UVA) can be obtained or, in a case in which the silicon oxide-coated zinc oxide is applied to cosmetic materials, a sticky feeling or a squeak feeling is suppressed, and favorable gluing, transparency, or the like can be developed. Furthermore, the cosmetic materials have appropriate shielding properties and thus become capable of covering skin spots, wrinkles, and the like. Therefore, an effect of improving a feeling during use while maintaining transparency can be obtained.

Meanwhile, the "average primary particle diameter of the zinc oxide particles" in the present embodiment is a value obtained using the following method. That is, in a case in which the zinc oxide of the present embodiment is observed using a transmission electron microscope (TEM) or the like, a predetermined number of zinc oxide, for example, 200 particles or 100 particles are selected. In addition, the longest straight line portions (longest diameters) of the respective zinc oxide particles are measured as the primary particle diameters, and these measurement values are weight-averaged, thereby obtaining the average primary particle diameter.

The shape of the zinc oxide particle may be a spherical shape, a massive shape, a spindle shape, a flake shape, or the like and is not particularly limited, and is preferably any one of a spindle shape or a flake shape. The spindle shape has a strong effect of suppressing secondary agglomeration, and spindle-shaped zinc oxide particles can be blended in a state of being close to primary particles even in actual cosmetic materials. In addition, the flake shape facilitates the zinc oxide particles to be more densely clogged than the spherical shape, has a stronger shielding force against ultraviolet (UV) rays, suppresses secondary agglomeration, suppresses a squeak feeling, and improves a feeling during use (spread).

As these zinc oxide particles, zinc oxide particles on which an alumina treatment, a titania treatment, or the like, which are generally carried out in the cosmetic product field, may be used.

In the silicon oxide-coated zinc oxide of the present embodiment, the surfaces thereof may be surface-treated with a silicone resin.

When the silicon oxide-coated zinc oxide of the present embodiment is surface-treated with a silicone resin, the affinity of the silicon oxide-coated zinc oxide to an oil phase, particularly, a silicone oil, becomes high. As a result, it becomes easier to blend the silicon oxide-coated zinc oxide of the present embodiment into a water-in-oil (W/O) type or oil-in-water (O/W) type cosmetic material.

That is, when the silicon oxide-coated zinc oxide formed by treating the surface thereof with a silicone resin is blended into an oil phase so as to produce a water-in-oil type or oil-in-water type cosmetic material, it is possible to suppress the elution of zinc ions in the water-in-oil (W/O) type or oil-in-water (O/W) type cosmetic material.

The silicone resin used in the surface treatment is not particularly limited as long as the silicone resin can be used as cosmetic materials and can be selected as necessary. Examples of the silicone resin include methyl hydrogen polysiloxane, dimethyl polysiloxane, methicone, hydrogen dimethicone, triethoxysilylethyl polydimethylsiloxyethyl dimethicone, triethoxysilylethyl polydimethylsiloxyethyl hexyl dimethicone, (acrylate/tridecyl acrylate/triethoxysilylpropyl methacrylate/dimethicone methacrylate) copolymers, triethoxycaprylylsilane, and the like. These silicone resins may be used singly, a mixture of two or more silicone resins may be used, or a copolymer of these silicone resins may be used.

The amount of the surface treatment of the silicone resin in the surface treatment may be appropriately adjusted depending on an oil phase being used. The amount of the surface treatment of the silicone resin is, for example, preferably 1% by mass or more and 20% by mass or less and more preferably 3% by mass or more and 10% by mass or less in relation to the total mass of the silicon oxide-coated zinc oxide.

[Method for preparing silicon oxide-coated zinc oxide]

A method for preparing silicon oxide-coated zinc oxide of the present embodiment is the following method. That is, a zinc oxide water-based suspension including zinc oxide particles having an average primary particle diameter of more than 50 nm and 500 nm or less and a silicate alkali metal salt aqueous solution including a silicate alkali metal salt are mixed together so as to prepare a water-based suspension including the zinc oxide particles and the silicate alkali metal salt, and an acid is added to the water-based suspension so as to cause a reaction, and then the obtained reaction product is thermally treated at a temperature of 200°C or higher and lower than 600°C.

In a case in which an alkali metal being included in the silicate alkali metal salt and at least one selected from Mg, Ca, and Ba are substituted in the silicon oxide-coated zinc oxide, examples thereof include the following method.

In the above-described method for preparing silicon oxide-coated zinc oxide of the present embodiment, first, an acid is added to the water-based suspension including zinc oxide particles and a silicate alkali metal salt so as to cause a reaction. Next, an aqueous solution including at least one selected from the group consisting of magnesium salts, calcium salts, and barium salts is added to the reaction solution. Therefore, a step in which alkali metal ions derived from the silicate alkali metal salt are ion-exchanged with at least one kind of ions selected from the group consisting of Mg, Ca, and Ba is carried out. Next, a reaction product after the ion exchange is thermally treated at a temperature of 200°C or higher and lower than 600°C.

Next, the method for preparing silicon oxide-coated zinc oxide of the present embodiment will be described in detail.

In the preparation of the zinc oxide water-based suspension, zinc oxide particles having an average primary particle diameter of more than 50 nm and 500 nm or less and water are mixed together, then, the zinc oxide particles are uniformly dispersed in the water, thereby preparing a zinc oxide water-based suspension including the zinc oxide particles.

The content of the zinc oxide particles in the zinc oxide water-based suspension is preferably 20% by mass or more and 60% by mass or less and more preferably 25% by mass or more and 55% by mass or less in order to enhance the reactivity with the silicate alkali metal salt.

A method for dispersing the zinc oxide particles in the water is not particularly limited, and, for example, an ultrasonic dispersion method, a stirring method, or the like can be used.

Next, the zinc oxide water-based suspension and a silicate alkali metal salt aqueous solution including a silicate alkali metal salt are mixed and stirred together, thereby preparing a water-based suspension including zinc oxide particles and a silicate metal salt.

The silicate alkali metal salt aqueous solution preferably includes 50% by mass or more of water.

As a solvent of the silicate alkali metal salt aqueous solution, in addition to water, solvents other than water can be used.

The solvents other than water are not particularly limited; however, when compatibility with water is taken into account, hydrophilic solvents such as water-soluble monovalent alcohols or polyvalent alcohols are preferred.

The silicate alkali metal salt is not particularly limited, and, for example, at least one selected from the group consisting of sodium orthosilicate salts, potassium orthosilicate salts, sodium metasilicate salts, potassium metasilicate salts, and sodium silicate can be used.

The content of the silicate alkali metal salt in the silicate alkali metal salt aqueous solution is an amount of a silicate alkali metal salt which reaches 12% by mass to 50% by mass in terms of silicon oxide in the silicon oxide-coated zinc oxide.

Next, the water-based suspension is heated, and an acid such as hydrochloric acid is gradually added thereto while stirring the water-based suspension, thereby adjusting pH to 5 to 9.

After that, the mixture is left to stand for one to five hours and is reacted.

The temperature at which the water-based suspension is heated is not particularly limited, but is preferably 40°C or higher and 100°C or lower and more preferably 50°C or higher and 70°C or lower in consideration of the precipitation rate of silicon oxide.

This step precipitates silicon oxide on the surfaces of the zinc oxide particles being included in the water-based suspension.

In a case in which the alkali metal and at least one selected from the group consisting of Mg, Ca, and Ba are substituted, examples thereof include the following method.

An aqueous solution including at least one selected from the group consisting of magnesium salts, calcium salts, and barium salts is added to and stirred with the water-based suspension, the mixture is left to stand for one to five hours, thereby carrying out a substitution reaction.

The amount of at least one selected from the group consisting of magnesium salts, calcium salts, and barium salts added is preferably equal to or more than the sum of the molar equivalents of alkali metal ions derived from the silicate alkali metal salt and other alkali metal ions.

Examples of the magnesium salts include magnesium chloride, magnesium sulfate, magnesium nitrate, and the like.

Examples of the calcium salts include calcium chloride, calcium nitrate, and the like.

Examples of the barium salts include barium nitrate and the like.

For example, in a case in which sodium silicate (water glass or sodium silicate) is used as the silicate alkali metal salt, an aqueous solution including at least one selected from the group consisting of magnesium salts, calcium salts, and barium salts is added to and stirred with the water-based suspension, whereby alkali metal ions in the silicon oxide coating (for example, sodium ions ($Na^+$)) and at least one selected from the group consisting of magnesium ions ($Mg^{2+}$) derived from a magnesium salt, calcium ions ($Ca^{2+}$) derived from a calcium salt, and barium ions ($Ba^{2+}$) derived from a barium salt exchange ions with each other.

The alkali metal in the silicon oxide coating is easily freed, but magnesium, calcium, and barium included in the silicon oxide coating are rarely freed, and an ion exchange reaction proceeds.

Next, this reaction solution is separated into solid and liquid, and the obtained reaction product is cleaned using a solvent such as water. Furthermore, the reaction product is dried at approximately 100°C to 150°C, and furthermore, is thermally treated (calcinated) at 200°C or higher and lower than 600°C for one to five hours, thereby producing the silicon oxide-coated zinc oxide of the present embodiment.

"Composition containing silicon oxide-coated zinc oxide"

A composition containing silicon oxide-coated zinc oxide of the present embodiment comprises the silicon oxide-coated zinc oxide of the present embodiment and a solvent.

In the composition containing silicon oxide-coated zinc oxide of the present embodiment, the average particle diameter of the silicon oxide-coated zinc oxide is preferably more than 50 nm and 2,000 nm or less, more preferably 100 nm or more and 500 nm or less, and still more preferably 200 nm or more and 300 nm or less.

Here, the reasons for setting the average particle diameter of the silicon oxide-coated zinc oxide to the above-described range are as described below. When the average particle diameter is 50 nm or less, the particle sizes of zinc oxide particles being included are also small, and there is a concern that it may become impossible to suppress the elution of zinc ions. On the other hand, when the average particle diameter exceeds 2,000 nm, in a case in which the silicon oxide-coated zinc oxide is used for cosmetic materials and the like, there is a concern that squeaks and the like may be caused and a feeling during use may deteriorate.

The content rate of the silicon oxide-coated zinc oxide in the composition containing silicon oxide-coated zinc oxide of the present embodiment may be appropriately adjusted in order to obtain desired ultraviolet ray-screening performance, and is not particularly limited, but the content rate thereof is preferably in a range of 1% by mass or more and 80% by mass or less, more preferably in a range of 20% by mass or more and 70% by mass or less, and still more preferably in a range of 30% by mass or more and 60% by mass or less.

Here, the content rate of the silicon oxide-coated zinc oxide preferably set to 1% by mass or more and 80% by mass or less. The reasons therefor are as described below. When the content rate of the silicon oxide-coated zinc oxide is less than 1% by mass, the composition containing silicon oxide-coated zinc oxide becomes incapable of exhibiting sufficient ultraviolet ray-screening performance, consequently, when the composition containing silicon oxide-coated zinc oxide is blended into a cosmetic material or the like, it is necessary to add a large amount of the composition containing silicon oxide-coated zinc oxide in order to exhibit desired ultraviolet ray-screening performance, and there is a concern that the preparation cost may become high, which is not preferable. On the other hand, when the content rate of the silicon oxide-coated zinc oxide exceeds 80% by mass, the viscous property of the composition containing silicon oxide-coated zinc oxide increases, and thus the dispersion stability of the silicon oxide-coated zinc oxide degrades, and there is a concern that the silicon oxide-coated zinc oxide may easily settle out, which is not preferable.

The solvent that is used in the composition containing silicon oxide-coated zinc oxide of the present embodiment is not particularly limited as long as the solvent is capable of dispersing the silicon oxide-coated zinc oxide.

As the solvent, for example, water, alcohols such as methanol, ethanol, 1-propanol, 2-propanol, 1-butanol, 2-butanol, octanol, and glycerin,

esters such as ethyl acetate, butyl acetate, ethyl lactate, propylene glycol monomethyl ether acetate, propylene glycol monoethyl ether acetate, and γ-butyrolactone; and

ethers such as diethyl ether, ethylene glycol monomethyl ether (methyl cellosolve), ethylene glycol monoethyl ether (ethyl cellosolve), ethylene glycol monobutyl ether (butyl cellosolve), diethylene glycol monomethyl ether, and diethylene glycol monoethyl ether can be preferably used.

In addition, as additional solvent that is used in the composition containing silicon oxide-coated zinc oxide of the present embodiment, ketones such as acetone, methyl ethyl ketone, methyl isobutyl ketone, acetyl acetone, and cyclohexanone;

aromatic hydrocarbons such as benzene, toluene, xylene, and ethyl benzene;

cyclic hydrocarbons such as cyclohexane;

amides such as dimethylformamide, N,N-dimethylacetoacetamide, and N-methyl pyrrolidone; and

chain-like polysiloxanes such as dimethyl polysiloxane, methyl phenyl polysiloxane, and diphenyl polysiloxane can also be preferably used.

In addition, cyclic polysiloxanes such as octamethylcyclotetrasiloxane, decamethylcyclopentasiloxane, and dodecamethylcyclohexanesiloxane; and

modified polysiloxanes such as amino-modified polysiloxane, polyether-modified polysiloxane, alkyl-modified polysiloxane, and fluorine-modified polysiloxane can also be preferably used.

These may be used singly, or a mixture of two or more solvents may be used.

The pH of the composition containing silicon oxide-coated zinc oxide of the present embodiment may be adjusted to 6 to 7 by adding a weak acid that is used in cosmetic materials such as citric acid or a pH buffer solution thereto. When the above-described weak acid is added thereto, it is possible to maintain the pH of the composition at 7 or less for a long period of time.

The composition containing silicon oxide-coated zinc oxide of the present embodiment may include ordinarily-used additives such as a dispersant, a stabilizer, a water-soluble binder, and a viscosity improver as long as the characteristics thereof are not impaired.

As the dispersant, an anionic surfactant, a cationic surfactant, an ampholytic surfactant, a non-ionic surfactant, a silane coupling agent such as an organoalkoxysilane or organochlorosilane, or a modified silicone such as a polyether-modified silicone or an amino-modified silicone is preferably used. The kind and amount of the dispersant may be appropriately selected depending on the particle diameter of composite particles and the kind of the target dispersion medium, and the dispersant may be used singly or a mixture of two or more dispersants may be used.

As the water-soluble binder, a polyvinyl alcohol (PVA), polyvinyl pyrrolidone, hydroxycellulose, polyacrylic acid, or the like can be used.

Regarding the viscosity improver, in a case in which the composition containing silicon oxide-coated zinc oxide of the present embodiment is applied to a cosmetic material, there is no particular limitation as long as the viscosity improver can be used for cosmetic materials. As the viscosity improver, for example, natural water-soluble macromolecules such as gelatin, casein, collagen, hyaluronic acid, albumin, and starch, semisynthetic macromolecules such as methyl cellulose, ethyl cellulose, methylhydroxypropyl cellulose, carboxymethyl cellulose, hydroxymethyl cellulose, hydroxypropyl cellulose, sodium carboxymethyl cellulose, and alginic acid propylene glycol ester, synthetic macromolecules such as polyvinyl alcohol, polyvinyl pyrrolidone, carbomers (carboxyvinyl polymer), polyacrylate, and polyethylene oxide, and inorganic minerals such as bentonite, laponite, and hectorite are preferably used. The viscosity improvers may be used singly or a combination of two or more viscosity improvers may be used.

Among these viscosity improvers, the synthetic macromolecule is preferred, and a carbomer (carboxyvinyl polymer) is more preferred.

Here, in a case in which a carbomer is used as the viscosity improver, the content rate of the carbomer in the composition containing silicon oxide-coated zinc oxide of the present embodiment is preferably in a range of 0.01% by mass or more and 10% by mass or less and more preferably in a range of 0.01% by mass or more and 3% by mass or less.

When the content rate of the carbomer in the composition containing silicon oxide-coated zinc oxide of the present embodiment is lower than 0.01% by mass, there is a concern that it may become impossible to obtain a viscosity-improving effect, and, on the other hand, when the content rate of the carbomer exceeds 10% by mass, the viscosity of the composition containing silicon oxide-coated zinc oxide becomes too high, and there are disadvantages that, in a case in which the composition containing silicon oxide-coated zinc oxide is applied to cosmetic materials, the spreading of cosmetic materials on the skin may become bad when the cosmetic materials are applied and spread onto the skin and the feeling during the use of the cosmetic materials may deteriorate, which is not preferable from the viewpoint of use.

In addition, in a case in which a carbomer is used as the viscosity improver, the hydrogen-ion exponent (pH) of the composition containing silicon oxide-coated zinc oxide is preferably in a range of 5 or more and 10 or less, more preferably in a range of 6 or more and 10 or less, and still more preferably in a range of 7 or more and 9 or less. When the pH of the composition containing silicon oxide-coated zinc oxide of the present embodiment is set in the above-described range, it is possible to suppress a change in the viscosity and the like over time.

Meanwhile, carbomers (carboxyvinyl polymer) are widely used as a viscosity improver for water-based cosmetic materials. But the carbomer improves the viscosity (gelatification) using the interaction between carboxyl groups or between carboxylate groups, and thus the presence of zinc ions breaks the network structure of the carbomer and disables the maintenance of a constant viscous property. Therefore, when several percent by mass of zinc oxide is mixed into an aqueous solution of a carbomer having an adjusted viscosity, the viscosity decreases within several hours. In addition, even in a case in which zinc oxide having a surface activity suppressed by coating the surface with an inorganic oxide or a resin is used, in many cases, the viscosity decreases or phases separate within several hours to several days. Therefore, in a case in which a carbomer and zinc oxide are jointly used, there is a requirement to suppress or reduce a decrease in the viscosity of a mixture including a carbomer and zinc oxide.

In addition, in a case in which a decrease in the viscosity of an aqueous solution of a carbomer is suppressed using zinc oxide having a surface activity suppressed by coating the surface with an inorganic oxide or a resin of the related art, there is a frequent significant problem of a decrease in the viscosity after a certain period of time elapses rather than a decrease in the viscosity in the initial phase.

The decrease in the viscosity in the initial phase can be coped with by, for example, setting the viscosity of the aqueous solution of a carbomer to be high in advance. However, when the viscosity changes in the middle to long term after a certain period of time elapses, the properties of cosmetic materials change during the distribution of the cosmetic materials, and the aging stability is impaired. Particularly, zinc oxide having a surface treated with an inorganic oxide or a resin has a certain degree of an elution-suppressing effect, and thus there has been a concern that zinc ions may be gradually eluted in the middle to long term.

Here, when the silicon oxide-coated zinc oxide of the present embodiment is used, in the composition containing silicon oxide-coated zinc oxide of the present embodiment, silicon oxide-coated zinc oxide having a stronger zinc ion elution-suppressing effect than zinc oxide coated with an inorganic oxide or a resin of the related art is used. Therefore, in the composition containing silicon oxide-coated zinc oxide of the present embodiment, even when a carbomer is used as the viscosity improver, the viscosity decreases only slightly over time, and the product stability of the composition becomes excellent.

In the composition containing silicon oxide-coated zinc oxide of the present embodiment, the value obtained by dividing the viscosity under acceleration conditions, for example, the viscosity of the composition after 300 hours of being stored at 40°C, by the viscosity that has decreased in the initial conditions, for example, the viscosity after 15 hours is preferably in a range of 0.8 or more and 1.2 or less.

As described above, when the value obtained by dividing the viscosity under acceleration conditions, that is, after 300 hours by the viscosity that decreases in the initial phase is set in the above-described range, it is possible to maintain the viscosity of the composition containing silicon oxide-coated zinc oxide of the present embodiment in the middle to long term.

In the composition containing silicon oxide-coated zinc oxide of the present embodiment, in a case in which the content rate of the silicon oxide-coated zinc oxide is set to 15% by mass and a 14 μm-thick coating is formed using the composition, the transmission of the coating of light having a wavelength of 450 nm is preferably 40% or higher, more preferably 45% or higher, and still more preferably 50% or higher.

The transmission can be obtained by applying the composition containing silicon oxide-coated zinc oxide which contains 15% by mass of the silicon oxide-coated zinc oxide onto a silica substrate using a bar coater so as to form a 14 μm-thick coating, and measuring the spectral transmission of the coating using a SPF analyzer UV-1000S (manufactured by Labsphere, Inc.) .

In addition, the light absorption spectrum is calculated from this transmission, the light absorption spectrum is integrated toward long wavelength sides from 290 nm, and a wavelength at which the integrated area reaches 90% of the entire integrated area from 290 to 400 nm can be calculated as a critical wavelength. As the critical wavelength increases, the shielding performance of UVA is higher, and thus the critical wavelength is preferably 375 nm or longer, more preferably 378 nm or longer, and still more preferably 380 nm or longer.

The method for preparing the composition containing silicon oxide-coated zinc oxide of the present embodiment is not particularly limited as long as the silicon oxide-coated zinc oxide can be dispersed in the above-described solvent.

As a dispersion method used for the above-described dispersion, a dispersion method in which a well-known dispersion device is used can be used. As the disperser, for example, dispersion methods in which, in addition to a stirrer, a beads mill in which zirconia beads are used, a ball mill, a homogenizer, an ultrasonic disperser, a kneader, a three roll mill, a rotation-revolution mixer, or the like is used are preferably used.

The time necessary for a dispersion treatment needs to be a sufficient time for the silicon oxide-coated zinc oxide to be uniformly dispersed in the solvent.

Next, as specific examples of the composition containing silicon oxide-coated zinc oxide of the present embodiment, (a) a silicone resin-based composition containing silicon oxide-coated zinc oxide in which the silicon oxide-coated zinc oxide is dispersed in a silicone resin that is a non-water-soluble dispersion medium and (b) a water-based composition containing silicon oxide-coated zinc oxide in which the silicon oxide-coated zinc oxide is dispersed in water will be respectively described.

(Silicone resin-based composition containing silicon oxide-coated zinc oxide)

The silicone resin-based composition containing silicon oxide-coated zinc oxide is a silicone resin-based composition in which the silicon oxide-coated zinc oxide is dispersed in a silicone resin. In this composition, the content rate of the silicon oxide-coated zinc oxide is set to 1% by mass or more and 80% by mass or less, more preferably set to 20% by mass or more and 70% by mass or less, and still more preferably set to 30% by mass or more and 60% by mass or less.

The average dispersed-particle diameter in the silicone resin-based composition containing silicon oxide-coated zinc oxide is preferably 60 nm or more and 2 $\mu$m or less, more preferably 80 nm or more and 800 nm or less, and still more preferably 100 nm or more and 500 nm or less.

The silicon oxide-coated zinc oxide may have a surface that is surface-treated with a silicone resin.

When the silicon oxide-coated zinc oxide is surface-treated with a silicone resin, the affinity to oil phases, particularly, silicone oil improves. Therefore, it becomes easier to blend the silicon oxide-coated zinc oxide into water-in-oil type (W/O type) or oil-in-water (O/W) type cosmetic materials.

That is, when the silicon oxide-coated zinc oxide obtained by carrying out a surface treatment with a silicone resin is blended into an oil phase, thereby producing a water-in-oil type or oil-in-water type cosmetic material, it is possible to suppress the elution of zinc ions in the water-in-oil type (W/O type) or oil-in-water (O/W) type cosmetic material.

The silicone resin being used for the surface treatment is not particularly limited as long as the silicone resin can be used as a cosmetic material. Examples of the silicone resin include methyl hydrogen polysiloxane, dimethyl polysiloxane, methicone, hydrogen dimethicone, triethoxysilylethyl polydimethylsiloxyethyl dimethicone, triethoxysilylethyl polydimethylsiloxyethyl hexyl dimethicone, (acrylate/tridecyl acrylate/triethoxysilylpropyl methacrylate/dimethicone methacrylate) copolymers, triethoxycaprylylsilane, and the like. These silicone resins may be used singly, a mixture of two or more silicone resins may be used, or a copolymer of these silicone resins may be used.

The silicone resin is not particularly limited as long as the silicone resin is a cyclic silicone resin or a straight silicone resin having a structural skeleton represented by following Formula (2).

$$(-(Si(CH_3)_2O-)_x \cdots \qquad (2)$$

(In Formula (2), X is in a range of 1 to 2,000.)

In this silicone resin, the value of X is preferably set in the above-described range since it becomes easy to mix the silicone resin with the silicon oxide-coated zinc oxide.

Examples of the silicone resin include chain-like siloxanes such as dimethyl polysiloxane, methyl phenyl polysiloxane, diphenyl polysiloxane, and methyl hydrogen polysiloxane, cyclic siloxanes such as hexamethylcyclotrisiloxane, octamethylcyclotetrasiloxane, decamethyl pentasiloxane, decamethylcyclopentasiloxane, dodecamethylcyclohexanesiloxane, and tetramethyltetrahydrogen polysiloxane, modified silicones such as amino-modified silicone, polyether-modified silicone, and alkyl-modified silicone, methyl trimethicone, and the like. These silicone resins may be used singly or a mixture of two or more silicone resins may be used.

The silicone resin-based composition containing silicon oxide-coated zinc oxide may include a dispersant.

Examples of the dispersant include modified silicones such as polyether-modified silicone, polyglycerin-modified silicone, amino-modified silicone, phenyl-modified silicone, alkyl-modified silicone, carbinol-modified silicone, and dimethyl silicone; surfactants such as an anionic surfactant, a cationic surfactant, an ampholytic surfactant, and a non-ionic surfactant; and silane coupling agents such as an organoalkoxysilane and organochlorosilane. These dispersants may be used singly or a mixture of two or more dispersants may be used.

The amount of the dispersant added is preferably in a range of 1% by mass or more and 50% by mass or less in relation to the mass of the silicon oxide-coated zinc oxide in the silicone resin-based composition containing silicon oxide-coated zinc oxide.

When the amount of the dispersant added is adjusted to be in the above-described range, even in a case in which the silicone resin-based composition containing silicon oxide-coated zinc oxide is used singly or is directly mixed into a cosmetic material, transparency can be sufficiently ensured in a case in which the composition is applied and spread onto the skin.

In addition, into the silicone resin-based composition containing silicon oxide-coated zinc oxide, a natural oil, a moisturizing agent, a viscosity improver, a perfume, a preservative, and the like may be further mixed as long as the characteristics of the composition are not impaired.

The silicone resin-based composition containing silicon oxide-coated zinc oxide may also be made into an oil phase, be emulsified with an aqueous component, and thus be made into an emulsified composition.

The oil phase preferably contains one of a higher alcohol and a higher fatty acid and more preferably contains both a higher alcohol and a higher fatty acid. When these components are contained in the oil phase, a firm skin feeling and a moisturized feeling improve, and the sustainability of these effects improves.

As the higher fatty acid, a saturated or unsaturated fatty acid having 12 to 24 carbon atoms is preferably used. As the above-described fatty acid, for example, myristic acid, palmitic acid, stearic acid, isostearic acid, linoleic acid, arachidonic acid, and the like are preferably used. These fatty acids may be used singly or a mixture of two or more higher fatty acids may be used.

In addition, into the oil phase, an oil-soluble preservative, an ultraviolet absorber, an oil-soluble chemical, an oil-soluble pigment, an oil-soluble protein, a vegetable oil, an animal oil, and the like may be appropriately mixed as necessary.

The method for preparing the silicone resin-based composition containing silicon oxide-coated zinc oxide is not particularly limited as long as it is possible to disperse the silicon oxide-coated zinc oxide in the silicone resin.

As a dispersion method that is used for the above-described dispersion, dispersion methods in which a well-known dispersion device can be used. As the dispersion device, for example, a stirrer, a beads mill, a ball mill, a homogenizer, an ultrasonic disperser, a kneader, a three roll mill, a rotation-revolution mixer, or the like can be preferably used.

The time necessary for a dispersion treatment needs to be a sufficient time for the silicon oxide-coated zinc oxide to be uniformly dispersed in the silicone resin.

(Water-based composition containing silicon oxide-coated zinc oxide)

The water-based composition containing silicon oxide-coated zinc oxide is a water-based composition formed by dispersing the silicon oxide-coated zinc oxide in a water-based dispersion medium containing an alcohol. In this composition, the content rate of the silicon oxide-coated zinc oxide is preferably 1% by mass or more and 80% by mass or less, more preferably 20% by mass or more and 70% by mass or less, and still more preferably 30% by mass or more and 60% by mass or less. In addition, the content of the water-based dispersion medium containing an alcohol in this composition is preferably 20% by mass to 99% by mass, more preferably 30% by mass to 80% by mass, and still more preferably 40% by mass to 70% by mass.

The average dispersed-particle diameter of the water-based composition containing silicon oxide-coated zinc oxide is preferably 60 nm or more and 2 $\mu$m or less, more preferably 80 nm or more and 800 nm or less, and still more preferably 100 nm or more and 500 nm or less.

Here, the water-based dispersion medium containing an alcohol is a dispersion medium including an alcohol and water. Examples of the alcohol include monovalent or polyvalent alcohols having 1 to 6 carbon atoms such as ethanol, 1-propanol, 2-propanol, 1-butanol, 2-butanol, octanol, glycerin, 1,3-butylene glycol, propylene glycol, and sorbitol. Among these, monovalent alcohols are preferred, and ethanol is particularly preferred.

In a case in which the water-based composition containing silicon oxide-coated zinc oxide is made up of the silicon oxide-coated zinc oxide and the water-based dispersion medium including the alcohol, the content rate of the alcohol is preferably in a range of 5% by mass or more and 20% by mass or less, and more preferably in a range of 10% by mass or more and 20% by mass or less.

Particularly, in a case in which the content rate of the alcohol is set in a range of 5% by mass or more and 20% by mass or less, it is possible to improve the dispersibility and the aging stability of the silicon oxide-coated zinc oxide in the water-based composition, which is preferable.

The water-based composition containing silicon oxide-coated zinc oxide may further include a water-soluble macromolecule in a range of 0.001% by mass or more and 10% by mass or less, more preferably in a range of 0.005% by mass or more and 5% by mass or less, and still more preferably in a range of 0.01% by mass or more and 3% by mass or less. In this case, it is necessary to adjust the content rates of the respective components so that the total of the respective content rates of the silicon oxide-coated zinc oxide, the water-based dispersion medium including an alcohol, and the water-soluble macromolecule does not exceed 100% by mass.

In a case in which the water-based composition containing silicon oxide-coated zinc oxide is applied to a cosmetic material, the water-soluble macromolecule included in the water-based composition is not particularly limited as long as the macromolecule can be used in cosmetic material use. Examples of the water-soluble macromolecule include gum arabic, sodium alginate, casein, carrageenan, galactan, carboxyvinyl polymers, carboxymethyl cellulose, sodium carboxymethyl cellulose, carboxymethyl starch, agar, xanthan gum, quince seed, guar gum, collagen, gelatin, cellulose, dextran, dextrin, tragacanth gum, hydroxyethyl cellulose, hydroxypropyl cellulose, sodium hyaluronate pectin, pullulan, methyl cellulose, and methylhydroxypropyl cellulose. These water-soluble macromolecules may be used singly, or a mixture of two or more water-soluble macromolecules may be used.

The water-soluble macromolecule plays roles as a dispersant and a viscosity adjuster, and, when added to the water-based composition, also improves the dispersibility and the aging stability of the silicon oxide-coated zinc oxide in the water-based composition containing silicon oxide-coated zinc oxide.

In a case in which the water-based composition containing silicon oxide-coated zinc oxide includes the water-soluble macromolecule, the content rate of the alcohol is preferably in a range of 5% by mass or more and 20% by mass or less and more preferably in a range of 15% by mass or more and 20% by mass or less.

Here, the content rate of the alcohol is set to 5% by mass or more and 20% by mass or less in a case in which the water-based composition containing silicon oxide-coated zinc oxide includes the water-soluble macromolecule. The reasons therefor are as described below. When the content rate thereof is lower than 5% by mass, the content of the alcohol is too small, and thus the water-soluble macromolecule cannot uniformly infiltrate into the alcohol and unevenly swells due to moisture . As a result, the dispersibility of the silicon oxide-coated zinc oxide degrades, handling becomes difficult, and furthermore, the aging stability of the water-based composition containing silicon oxide-coated zinc oxide degrades, which is not preferable. In addition, when the content rate of the alcohol exceeds 20% by mass, the viscous property of the entire water-based composition containing silicon oxide-coated zinc oxide becomes high. As a result, the dispersion stability of the silicon oxide-coated zinc oxide degrades, and the aging stability of the water-based composition containing silicon oxide-coated zinc oxide also degrades, which is not preferable.

The water-based composition containing silicon oxide-coated zinc oxide can be obtained by mixing the silicon oxide-coated zinc oxide into the water-based dispersion medium including the alcohol or the water-based dispersion medium including the alcohol and the water-soluble macromolecule, and then mixing water into the mixture so as to disperse the above-described components. The amount of water in the composition may be appropriately adjusted and is preferably in a range of 15% by mass or more and 94% by mass or less in consideration of the dispersion stability and aging stability of the silicon oxide-coated zinc oxide.

When the amount of water is adjusted to be in the above-described range, it is possible to obtain a water-based composition containing silicon oxide-coated zinc oxide in which, even in a case in which the composition is used singly or is mixed into a cosmetic material, transparency can be sufficiently ensured in a case in which the composition is applied and spread onto the skin.

The silicon oxide-coated zinc oxide-containing water-based composition may also be an emulsified composition which has been mixed with an oil phase.

[Cosmetic material]

A cosmetic material of the present embodiment comprises at least one of the above-described silicon oxide-coated zinc oxide and the above-described composition containing silicon oxide-coated zinc oxide in a base.

In a case in which the silicon oxide-coated zinc oxide is used in ultraviolet ray-screening use, the average dispersed-particle diameter of this silicon oxide-coated zinc oxide is preferably 60 nm or more and 2 $\mu$m or less, more preferably 80 nm or more and 800 nm or less, and still more preferably 100 nm or more and 500 nm or less.

The content rate of the silicon oxide-coated zinc oxide included in the cosmetic material for which at least one of the above-described silicon oxide-coated zinc oxide and the above-described composition containing silicon oxide-coated zinc oxide is used may be appropriately adjusted. The content rate of silicon oxide-coated zinc oxide is preferably 1% by mass or more and 60% by mass or less in relation to the mass of the entire cosmetic material. When the content of the silicon oxide-coated zinc oxide is in the above-described range, transparency can be sufficiently ensured, and furthermore, a cosmetic material having no rough feeling and the like and providing an excellent feeling during use can be obtained.

The cosmetic material of the present embodiment may include organic ultraviolet ray-screening agents, inorganic ultraviolet ray-screening agents, additives, and the like as long as the effects of the present invention are not impaired.

Examples of the organic ultraviolet ray-screening agents include anthranilates, cinnamic acid derivatives, salicylic acid derivatives, camphor derivatives, benzophenone derivatives, $\beta,\beta$'-diphenylacrylate derivatives, benzotriazole derivatives, benzalmalonate derivatives, benzimidazole derivatives, imidazolines, bisbenzoazolyl derivatives, p-amino benzoic acid (PABA) derivatives, and methylene bis(hydroxyphenyl benzotriazole) derivatives, and it is possible to selectively use at least one selected from the above-described group.

In addition, examples of the inorganic ultraviolet ray-screening agents include oxides other than zinc oxide, for example, titanium oxide and cerium oxide, and it is possible to appropriately select and use at least one selected from the above-described group.

The cosmetic material of the present embodiment can be obtained by blending the silicon oxide-coated zinc oxide into a base such as an emulsion, a cream, a foundation, a lipstick, rouge, or eyeshadow as in the related art.

Furthermore, it is possible to obtain a water-based cosmetic material having excellent ultraviolet ray-screening performance, transparent feeling, and feeling during use by blending the silicon oxide-coated zinc oxide into a water-based cosmetic material such as a facial lotion or a sunscreen for which formulation is difficult in the related art.

Additionally, when this cosmetic material is used as a component of cosmetic products, it is possible to provide a variety of cosmetic products such as skincare cosmetic products, makeup cosmetic products, and bodycare cosmetic products having excellent ultraviolet shielding performance, transparency, and a feeling during use. Particularly, the cosmetic material is preferred for sunscreens as bodycare cosmetic products requiring ultraviolet shielding performance.

As described above, according to the silicon oxide-coated zinc oxide of the present embodiment, it is possible to suppress the outward elution of zinc ions. Therefore, in a case in which the silicon oxide-coated zinc oxide of the present embodiment is applied to cosmetic materials, it is possible to suppress the degradation of performance, discoloration, changes in viscosity, and the like as cosmetic materials, caused by the elution of zinc ions. In addition, it is possible to provide silicon oxide-coated zinc oxide suitable for weakly acidic formulations.

In addition, in a case in which the silicon oxide-coated zinc oxide of the present embodiment includes at least one selected from the group consisting of Mg, Ca, and Ba, it is possible to further suppress changes in pH when the silicon oxide-coated zinc oxide is mixed with pure water.

According to the method for preparing silicon oxide-coated zinc oxide of the present embodiment, a zinc oxide water-based suspension including zinc oxide particles having an average primary particle diameter of more than 50 nm and 500 nm or less is mixed with a silicate alkali metal salt aqueous solution including a silicate alkali metal salt so as to prepare a water-based suspension including the zinc oxide particles and the silicate alkali metal salt, an acid is added to this water-based suspension so as to cause a reaction, and then a thermal treatment is carried out. Therefore, even in a case in which silicon oxide-coated zinc oxide is applied to water-based materials such as water-based cosmetic materials, it is possible to suppress the elution of zinc ions. As a result, it is possible to easily produce at a low price silicon oxide-coated zinc oxide capable of maintaining the stability of the qualities of water-based materials.

According to the composition containing silicon oxide-coated zinc oxide of the present embodiment, since the silicon oxide-coated zinc oxide of the present embodiment is included, it is possible to suppress the outward elution of zinc elements being included in the silicon oxide-coated zinc oxide of the present embodiment in a form of zinc ions. Therefore, it is possible to suppress the degradation of performance, discoloration, changes in viscosity, and the like as compositions, caused by the elution of zinc ions.

According to the cosmetic material of the present embodiment, since at least one of the silicon oxide-coated zinc oxide and the composition containing silicon oxide-coated zinc oxide of the present embodiment is included in the base, it is possible to suppress the outward elution of zinc elements being included in the silicon oxide-coated zinc oxide of the present embodiment in a form of zinc ions. Therefore, it is possible to suppress the degradation of performance, discoloration, changes in viscosity, and the like as cosmetic materials, caused by the elution of zinc ions.

Examples

Hereinafter, the present invention will be more specifically described using Examples 1 to 6 and Comparative Examples 1 to 9, but the present invention is not limited to the following examples.

[Example 1]

[Silicon oxide-coated zinc oxide]

Zinc oxide particles (having an average particle diameter of 250 nm, manufactured by Sumitomo Osaka Cement Co., Ltd.) and water were mixed together, and then ultrasonic dispersion was carried out, thereby preparing a zinc oxide water-based suspension in which the content rate of zinc oxide was 50% by mass.

Next, this zinc oxide water-based suspension was added to and stirred with a sodium silicate aqueous solution including sodium silicate in an amount of 17.7% by mass of the mass of the zinc oxide particles in the zinc oxide water-based suspension in terms of silicon oxide (the content of silicon oxide in silicon oxide-coated zinc oxide was 15% by mass), thereby producing a suspension.

Next, this suspension was heated to 60°C, dilute hydrochloric acid was slowly added thereto while stirring the suspension, thereby adjusting the pH to 6. After that, the mixture was left to stand for 2 hours, the same mass of a calcium chloride aqueous solution (50% by mass of calcium chloride dihydrate) as the mass of the zinc oxide particles in this suspension was added to and stirred with the mixture, and furthermore, the mixture was left to stand for 2 hours.

Next, this suspension was separated into solid and liquid using a centrifugal separator, and the obtained solid was cleaned with water. After that, this solid was dried at 150°C and, furthermore, was thermally treated (calcinated) at 500°C for 3 hours, thereby producing silicon oxide-coated zinc oxide of Example 1.

As a result of measuring the contents of Na and Ca in the silicon oxide-coated zinc oxide of Example 1 by means of an atomic absorption analysis, the content of Na was 0.12% by mass, and the content of Ca was 0.10% by mass.

The NMR spectrum of the silicon oxide-coated zinc oxide of Example 1 was measured by means of MAS-nuclear magnetic resonance (NMR) spectroscopy in which solid $^{29}Si$ is used, and the area ratios $Q^0$, $Q^1$, $Q^2$, $Q^3$, and

$Q^4$ of signals attributed to individual environments of $Q^0$, $Q^1$, $Q^2$, $Q^3$, and $Q^4$ were calculated from the peak area ratios of the NMR spectrum.

The abundance ratio of silicon in the silicon oxide coating in a $Q^3$ environment was indicated by $Q^3$, the abundance ratio in a $Q^4$ environment was indicated by $Q^4$, and the value of $Q^3+Q^4$ and the value of $Q^4/(Q^3+Q^4)$ were calculated. As a result, $Q^3+Q^4 \geq 0.6$ was satisfied, and $Q^4/(Q^3+Q^4) < 0.5$ was satisfied.

[Example 2]

[Silicon oxide-coated zinc oxide]

Zinc oxide particles (having an average particle diameter of 250 nm, manufactured by Sumitomo Osaka Cement Co., Ltd.) and water were mixed together, and then ultrasonic dispersion was carried out, thereby preparing a zinc oxide water-based suspension in which the content rate of zinc oxide was 30% by mass.

Next, this zinc oxide water-based suspension was added to and stirred with a sodium silicate aqueous solution including sodium silicate in an amount of 17.7% by mass of the mass of the zinc oxide particles in the zinc oxide water-based suspension in terms of silicon oxide (the content of silicon oxide in silicon oxide-coated zinc oxide was 15% by mass), thereby producing a suspension.

Next, this suspension was heated to 60°C, dilute hydrochloric acid was slowly added thereto while stirring the suspension, thereby adjusting the pH to 6. After that, the mixture was left to stand for 2 hours, the same mass of a calcium chloride aqueous solution (50% by mass of calcium chloride dihydrate) as the mass of the zinc oxide particles in this suspension was added to and stirred with the mixture, and furthermore, the mixture was left to stand for 2 hours.

Next, this suspension was separated into solid and liquid using a centrifugal separator, and the obtained solid was cleaned with water. After that, this solid was dried at 150°C and, furthermore, was thermally treated (calcinated) at 500°C for 3 hours, thereby producing silicon oxide-coated zinc oxide of Example 2.

[Example 3]

[Silicon oxide-coated zinc oxide]

Zinc oxide particles (having an average particle diameter of 250 nm, manufactured by Sumitomo Osaka Cement Co., Ltd.) and water were mixed together, and then ultrasonic dispersion was carried out, thereby preparing a zinc oxide water-based suspension in which the content rate of zinc oxide was 50% by mass.

Next, this zinc oxide water-based suspension was added to and stirred with a sodium silicate aqueous solution including sodium silicate in an amount of 17.7% by mass of the mass of the zinc oxide particles in the zinc oxide water-based suspension in terms of silicon oxide (the content of silicon oxide in silicon oxide-coated zinc oxide was 15% by mass), thereby producing a suspension.

Next, this suspension was heated to 60°C, dilute hydrochloric acid was slowly added thereto while stirring the suspension, thereby adjusting the pH to 6, and then the mixture was left to stand for 2 hours.

Next, this suspension was separated into solid and liquid using a centrifugal separator, and the obtained solid was cleaned with water. After that, this solid was dried at 150°C and, furthermore, was thermally treated (calcinated) at 500°C for 3 hours, thereby producing silicon oxide-coated zinc oxide of Example 3.

[Example 4]

[Silicon oxide-coated zinc oxide]

Zinc oxide particles (having an average particle diameter of 250 nm, manufactured by Sumitomo Osaka Cement Co., Ltd.) and water were mixed together, and then ultrasonic dispersion was carried out, thereby preparing a zinc oxide water-based suspension in which the content rate of zinc oxide was 30% by mass.

Next, this zinc oxide water-based suspension was added to and stirred with a sodium silicate aqueous solution including sodium silicate in an amount of 17.7% by mass of the mass of the zinc oxide particles in the zinc oxide water-based suspension in terms of silicon oxide (the content of silicon oxide in silicon oxide-coated zinc oxide was 15% by mass), thereby producing a suspension.

Next, this suspension was heated to 60°C, dilute hydrochloric acid was slowly added thereto while stirring the suspension, thereby adjusting the pH to 6, and then the mixture was left to stand for 2 hours.

Next, this suspension was separated into solid and liquid using a centrifugal separator, and the obtained solid was cleaned with water. After that, this solid was dried at 150°C and, furthermore, was thermally treated (calcinated) at 500°C for 3 hours, thereby producing silicon oxide-coated zinc oxide of Example 4.

[Comparative Example 1]

[Silicon oxide-coated zinc oxide]

Zinc oxide particles (having an average particle diameter of 250 nm, manufactured by Sumitomo Osaka Cement Co., Ltd.) and water were mixed together, and then ultrasonic dispersion was carried out, thereby preparing a zinc oxide water-based suspension in which the content rate of zinc oxide was 20% by mass.
Next, this zinc oxide water-based suspension was added to and stirred with a sodium silicate aqueous solution including sodium silicate in an amount of 5.3% by mass of the mass of the zinc oxide particles in the zinc oxide water-based suspension in terms of silicon oxide (the content of silicon oxide in silicon oxide-coated zinc oxide was 5% by mass), thereby producing a suspension.
Next, this suspension was heated to 60°C, dilute hydrochloric acid was slowly added thereto while stirring the suspension, thereby adjusting the pH to 6. After that, the mixture was left to stand for 2 hours, the same mass of a calcium chloride aqueous solution (50% by mass of calcium chloride dihydrate) as the mass of the zinc oxide particles in this suspension was added to and stirred with the mixture, and furthermore, the mixture was left to stand for 2 hours.
Next, this suspension was separated into solid and liquid using a centrifugal separator, and the obtained solid was cleaned with water. After that, this solid was dried at 150°C and, furthermore, was thermally treated (calcinated) at 500°C for 3 hours, thereby producing silicon oxide-coated zinc oxide of Comparative Example 1.

[Comparative Example 2]

[Silicon oxide-coated zinc oxide]

Zinc oxide particles (having an average particle diameter of 250 nm, manufactured by Sumitomo Osaka Cement Co., Ltd.) and water were mixed together, and then ultrasonic dispersion was carried out, thereby preparing a zinc oxide water-based suspension in which the content rate of zinc oxide was 20% by mass.
Next, this zinc oxide water-based suspension was added to and stirred with a sodium silicate aqueous solution including sodium silicate in an amount of 11.1% by mass of the mass of the zinc oxide particles in the zinc oxide water-based suspension in terms of silicon oxide (the content of silicon oxide in silicon oxide-coated zinc oxide was 10% by mass), thereby producing a suspension.
Next, this suspension was heated to 60°C, dilute hydrochloric acid was slowly added thereto while stirring the suspension, thereby adjusting the pH to 6. After that, the mixture was left to stand for 2 hours, the same mass of a calcium chloride aqueous solution (50% by mass of calcium chloride dihydrate) as the mass of the zinc oxide particles in this suspension was added to and stirred with the mixture, and furthermore, the mixture was left to stand for 2 hours.
Next, this suspension was separated into solid and liquid using a centrifugal separator, and the obtained solid was cleaned with water. After that, this solid was dried at 150°C and, furthermore, was thermally treated (calcinated) at 500°C for 3 hours, thereby producing silicon oxide-coated zinc oxide of Comparative Example 2.

[Comparative Example 3]

As silicon oxide-coated zinc oxide of Comparative Example 3, a commercially available product of SIH20-ZnO-650 (having an average particle diameter of 25 nm, a silica coating was formed using sodium silicate so that the content of silicon oxide in the silicon oxide-coated zinc oxide reached 15% by mass; manufactured by Sumitomo Osaka Cement Co., Ltd.) was used.
As a result of measuring the contents of Na and Ca in the silicon oxide-coated zinc oxide of Comparative Example 3 in the same manner as in Example 1, the content of Na was 0.57% by mass, and Ca was not detected.
In addition, $Q^0$, $Q^1$, $Q^2$, $Q^3$, and $Q^4$ were calculated in the same manner in Example 1. As a result, $Q^3+Q^4<0.6$ and $Q^4/(Q^3+Q^4)<0.5$.

[Comparative Example 4]

Zinc oxide in which no silica coatings were formed and the average particle diameter was 25 nm (manufactured by Sumitomo Osaka Cement Co., Ltd., ZnO-650) was used as Comparative Example 4.

[Comparative Example 5]

Zinc oxide having an average particle diameter of 250 nm (manufactured by Sumitomo Osaka Cement Co. , Ltd.) which was used in Example 1 was used as Comparative Example 5.

[Evaluation]

The respective silicon oxide-coated zinc oxides and the respective zinc oxides of Examples 1 to 4 and Comparative Examples 1 to 5 were evaluated.

Evaluation items are as described below.

(1) Elution ratio S in buffer solution having pH=5

0.015 g of silicon oxide-coated zinc oxide (or silicon oxide) was added to 0.05 mol/l of potassium hydrogen phthalate/NaOH buffer solution (pH=5), the components were stirred using a stirrer at 25°C for 1 hour and were separated into solid and liquid using a centrifugal separator, thereby obtaining a zinc extraction liquid.

Next, a droplet of a 0.1% xylenol orange solution was added to this zinc extraction liquid, and the mixture was titrated with 0.004 mol/l of an EDTA solution. The amount of zinc oxide eluted into the zinc extraction liquid was calculated from the amount of EDTA required for the titration.

Meanwhile, the amount of zinc oxide in the silicon oxide-coated zinc oxide was measured by means of an ICP analysis . Therefore, the calculation results of the elution ratio S of zinc oxide defined by following Expression (1) are shown in Table 1.

```
Elution ratio S=(amount of eluted zinc oxide)/(amount of

zinc oxide in silicon oxide-coated zinc oxide) ···      (1)
```

(2) Elution ratio in oleic acid

10 g of silicon oxide-coated zinc oxide (or zinc oxide) was added to and mixed with 50 g of an isopropyl alcohol solution including 20% by mass of oleic acid so as to produce a suspension, and the suspension was left to stand for 48 hours.

The fluidity of the suspension after being left to stand was visually checked, and results in which the fluidity was evaluated as A in the case of fluidic suspensions and as B in the case of non-fluidic suspensions are shown in Table 1.

Since, when eluted, zinc ions react with oleic acid so as to solidify the suspension, the case of A in which the suspension is fluidic means that the elution of zinc ions is suppressed. On the other hand, the case of B in which the suspension is not fluidic means that the elution of zinc ions is not suppressed.

(3) pH in pure water

10 g of silicon oxide-coated zinc oxide (or zinc oxide) was added to 90 g of pure water (pH=6 to 7), the mixture was stirred using a stirrer at 25°C for 20 hours so as to produce a suspension, and the pH of this suspension was measured. The results are shown in Table 1.

As the pH approximates to 6 to 7, the elution of zinc or sodium is suppressed, and it is indicated that quality stability when the suspension is blended into cosmetic materials is favorable.

[Table 1]

| | | Example 1 | Example 2 | Example 3 | Example 4 | Comparative Example 1 | Comparative Example 2 | Comparative Example 3 | Comparative Example 4 | Comparative Example 5 |
|---|---|---|---|---|---|---|---|---|---|---|
| Preparation conditions | Content of silicon oxide in silicon oxide-coated zinc oxide (% by mass) | 15 | 15 | 15 | 15 | 5 | 10 | 15 | 0 | 0 |
| | Concentration of zinc oxide in zinc oxide water-based suspension (% by mass) | 50 | 30 | 50 | 30 | 20 | 20 | - | - | - |
| | Calcium treatment | Yes | Yes | No | NO | Yes | Yes | No | NO | No |
| Evaluation | Elution ratio (%) of zinc oxide | 24 | 48 | 21 | 33 | 97 | 89 | 91 | 100 | 100 |
| | Elution of zinc ions into oleic acid | A | A | A | A | - | A | B | B | A |
| | pH in pure water | 7.9 | 6.9 | 8.1 | 7.1 | - | 6.8 | 9.3 | 7.6 | - |

EP 3 219 674 B1

18

(4) Stability of pH in neutral region by addition of citric acid

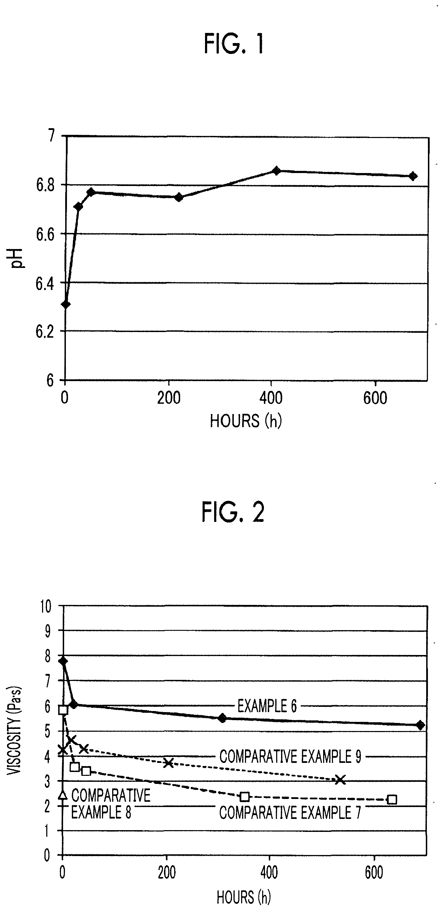

10 g of silicon oxide-coated zinc oxide of Example 1 was added to 90 g of an aqueous solution including 0.2% by mass of citric acid, and measurement results of changes in the pH of the suspension during stirring using a stirrer at 25°C are illustrated in FIG. 1.

From the results of FIG. 1, it was confirmed that the pH of the silicon oxide-coated zinc oxide of Example 1 can be controlled to be in a range of 6 to 7 by adding a small amount of an acid in a water-based environment.

(5) Photocatalytic activity

A 2-propanol aqueous solution in which the content rate of 2-propanol was adjusted to 300 ppm was produced. 0.01 g and 0.00 g (blank) of the silicon oxide-coated zinc oxides obtained in Example 1, and 0.01 g of the zinc oxide of Comparative Example 5 were respectively mixed into 9.99 g of the above-described 2-propanol aqueous solution and were ultrasonically dispersed, thereby producing two suspensions (Example 1 and Comparative Example 5) and a blank solution.

Next, 0.3 g of the ultrasonically-dispersed suspension or the blank solution was fed into a 10 mm×10 mm silica cell, and furthermore, 2.7 g of the 2-propanol aqueous solution was fed thereinto, thereby producing 3 g of a sample liquid.

The sample liquid was irradiated using an ultraviolet lamp (central wavelength: 254 nm) at an irradiation distance of 10 cm for 6 hours, and then a supernatant liquid was collected.

Next, the amount of acetone being included in the collected supernatant liquid was determined using gas chromatography.

As a result, in Example 1, the amount of acetone was 19 ppm and the amount of acetone in the blank was 19 ppm, and, in Comparative Example 5, the amount of acetone was 37 ppm.

From these results, it was confirmed that, in the silicon oxide-coated zinc oxide of Example 1, the amount of acetone being generated by the oxidation decomposition of 2-propanol was smaller than that in the zinc oxide of Comparative Example 5 and, furthermore, was the same as the amount of acetone in the blank, and the photocatalytic activity was suppressed.

[Example 5]

30 parts by mass of the silicon oxide-coated zinc oxide of Example 1, 4.5 parts by mass of polyether-modified silicone, and 65.5 parts by mass of decamethyl cyclopentanesiloxane (trade name: SH245, manufactured by Dow Corning Toray Co., Ltd.) (hereinafter, abbreviated as "D5") were mixed together.

Next, this mixed liquid was dispersed using a sand mill, thereby producing a composition containing silicon oxide-coated zinc oxide of Example 5.

The obtained composition containing silicon oxide-coated zinc oxide was diluted with D5 so that the concentration of zinc oxide reached 15% by mass. This diluted liquid was applied onto a silica substrate using a bar coater, thereby forming a 13.7 μm-thick coated film.

The light absorption spectrum of this coated film was measured using an SPF analyzer UV-1000S (manufactured by Labsphere Inc.), the measured light absorption spectrum was integrated toward long wavelength sides from 290 nm, and a wavelength at which the integrated area reached 90% of the entire integrated area from 290 to 400 nm was calculated as the critical wavelength. As a result, the critical wavelength was 380 nm.

[Comparative Example 6]

A composition containing silicon oxide-coated zinc oxide of Comparative Example 6 was produced in the same manner as in Example 5 except for the fact that the silicon oxide-coated zinc oxide of Comparative Example 3 was used instead of the silicon oxide-coated zinc oxide of Example 1.

A coated film was formed using the obtained composition containing silicon oxide-coated zinc oxide in the same manner as in Example 5, and the critical wavelength of the coated film was calculated to be 370 nm.

[Example 6]

1.5 g of carbomer Ultrez10 (manufactured by Nikko Chemicals Co., Ltd.) was added to pure water, and then 10% by mass of a sodium hydroxide aqueous solution was added dropwise so as to adjust the pH, thereby producing a carbomer gel which included 0.15% by mass of the carbomer and had a pH of 7.5.

Next, this carbomer gel and the silicon oxide-coated zinc oxide of Example 1 were mixed and stirred together so that the mass ratio reached 95:5, thereby producing a composition containing silicon oxide-coated zinc oxide of Example 6.

The viscosities of the obtained compositions containing silicon oxide-coated zinc oxide were measured using a viscosimeter BII-type viscometer (manufactured by Toki Sangyo Co., Ltd.) under conditions of 20°C and 30 rpm.

Next, this composition containing silicon oxide-coated zinc oxide was held at 40°C using a constant temperature tank, and the viscosity was measured every predetermined time under conditions of 20°C and 30 rpm. The viscosity change of this composition containing silicon oxide-coated zinc oxide is illustrated in FIG. 2.

[Comparative Example 7]

A composition containing silicon oxide-coated zinc oxide of Comparative Example 7 was obtained in the same manner as in Example 6 except for the fact that the silicon oxide-coated zinc oxide of Comparative Example 2 was used instead of the silicon oxide-coated zinc oxide of Example 1.

The viscosity change of this composition containing silicon oxide-coated zinc oxide was measured in the same manner as in Example 6. The result is illustrated in FIG. 2.

[Comparative Example 8]

A composition containing zinc oxide of Comparative Example 8 was obtained in the same manner as in Example 6 except for the fact that the zinc oxide of Comparative Example 4 was used instead of the silicon oxide-coated zinc oxide of Example 1.

The viscosity change of this composition containing zinc oxide was measured in the same manner as in Example 6. The result is illustrated in FIG. 2.

[Comparative Example 9]

A composition containing zinc oxide of Comparative Example 9 was obtained in the same manner as in Example 6 except for the fact that the zinc oxide of Comparative Example 5 was used instead of the silicon oxide-coated zinc oxide of Example 1.

The viscosity change of this composition containing zinc oxide was measured in the same manner as in Example 6. The result is illustrated in FIG. 2.

From the results of Table 1, it was confirmed that, in the silicon oxide-coated zinc oxide of Examples 1 to 4, the elution ratios of zinc ions in the buffer solution having a pH of 5 can be suppressed to 50% or less compared with the silicon oxide-coated zinc oxide or the zinc oxide of Comparative Examples 1 to 5.

Furthermore, it was confirmed that the silicon oxide-coated zinc oxide of Examples 1 to 4 is capable of maintaining the pH to be as neutral as 7 to 8 even when mixed with pure water.

In addition, from FIG. 1, it was confirmed that the silicon oxide-coated zinc oxide of Example 1 is enabled to maintain the pH of 7 or less which is more preferred for use for cosmetic materials by adding a small amount of citric acid to pure water.

From the comparison between Examples 1 to 4 and Comparative Examples 3 and 4, it was confirmed that, when the average primary particle diameter of zinc oxide exceeds 50 nm, the elution of zinc in oleic acid aqueous solutions can be suppressed.

In addition, from the comparison of the critical wavelengths of the compositions of Example 5 and Comparative Example 6, it was confirmed that the critical wavelength of the composition of Example 5 is longer than the critical wavelength of the composition of Comparative Example 6 and ultraviolet shielding properties are excellent.

In addition, from FIG. 2, it was confirmed that, when the composition containing silicon oxide-coated zinc oxide of Example 6, the composition containing silicon oxide-coated zinc oxide of Comparative Example 7, and the compositions containing zinc oxide of Comparative Examples 8 and 9 are compared with each other, in the composition containing silicon oxide-coated zinc oxide of Example 6, a decrease in the viscosity is suppressed even after 500 hours elapses, and the carbomer resistance is excellent.

In addition, from the comparison between Examples 1 and 3 and between Examples 2 and 4, it was confirmed that, the pH during the mixing with pure water can be further approximated to 7 by substituting sodium with calcium. That is, it was confirmed that, when sodium is substituted with calcium, the content of sodium decreases, the content of calcium increases, and a change of the pH toward the alkaline side is small.

Industrial Applicability

The silicon oxide-coated zinc oxide of the present invention, even in a case in which this silicon oxide-coated zinc oxide is applied to water-based material materials such as water-based cosmetic materials, is capable of suppressing the elution of zinc ions and suppressing changes in the hydrogen-ion exponent (pH) of water-based materials. Therefore, the silicon oxide-coated zinc oxide of the present invention is capable of maintaining the stability of the qualities of water-based materials and thus is capable of improving the degree of freedom in formulation in the case of being applied to water-based cosmetic products and is highly industrially valuable.

**Claims**

1. Silicon oxide-coated zinc oxide formed by coating surfaces of zinc oxide particles with a silicon oxide coating, wherein the amount of silicon oxide in the silicon oxide-coated zinc oxide is 12% by mass or more, an average primary particle diameter of the zinc oxide particles is more than 50 nm and 500 nm or less, the average primary particle diameter of the zinc oxide particles is a weight-averaged value of a primary particle diameter measured using a transmission electron microscope, and when held in a buffer solution having a pH of 5 for 1 hour, an elution ratio S of the zinc oxide defined by following Expression (1) is 50% or less :

$$\text{Elution ratio S=(amount of eluted zinc oxide)/(amount of zinc oxide in silicon oxide-coated zinc oxide)} \quad (1).$$

2. The silicon oxide-coated zinc oxide according to claim 1, wherein the average primary particle diameter of the zinc oxide particles is 200 nm or more and 500 nm or less.

3. The silicon oxide-coated zinc oxide according to claim 1 or 2, wherein the silicon oxide coating comprises at least one element selected from the group consisting of magnesium salts, calcium salts, and barium salts.

4. The silicon oxide-coated zinc oxide according to any one of claims 1 to 3, wherein the average primary particle diameter of the zinc oxide particles is 200 nm or more and 300 nm or less, and primary particle diameters of 90% or more of the zinc oxide particles are 100 nm or more and 400 nm or less.

5. A method for preparing silicon oxide-coated zinc oxide, comprising:

    mixing a zinc oxide water-based suspension including zinc oxide particles having an average primary particle diameter of more than 50 nm and 500 nm or less with a silicate alkali metal salt aqueous solution including a silicate alkali metal salt so as to prepare a water-based suspension including the zinc oxide particles and the silicate alkali metal salt, and adding an acid to the water-based suspension so as to cause a reaction; and thermally treating the obtained reaction product at a temperature of 200°C or higher and lower than 600°C for one to five hours.

6. The method for preparing silicon oxide-coated zinc oxide according to claim 5, wherein an aqueous solution including at least one selected from the group consisting of magnesium salts, calcium salts, and barium salts is added to and stirred with a reaction solution which was obtained by adding the acid, and the mixture is left to stand for one to five hours, thereby carrying out a substitution reaction.

7. A composition containing silicon oxide-coated zinc oxide comprising:
   the silicon oxide-coated zinc oxide according to any one of claims 1 to 4.

8. A cosmetic material comprising:
   at least one of the silicon oxide-coated zinc oxide according to any one of claims 1 to 4 and the composition containing silicon oxide-coated zinc oxide according to claim 7 in a base.

**Patentansprüche**

1. Mit Siliziumoxid beschichtetes Zinkoxid, gebildet durch Beschichten von Oberflächen von Zinkoxidteilchen mit einer Siliziumoxidbeschichtung, wobei die Menge an Siliziumoxid in dem mit Siliziumoxid beschichteten Zinkoxid 12 Massen-% oder mehr beträgt,

ein durchschnittlicher Primärteilchendurchmesser der Zinkoxidteilchen mehr als 50 nm und 500 nm oder weniger beträgt,

der durchschnittliche Primärteilchendurchmesser der Zinkoxidteilchen ein gewichtsgemittelter Wert eines Primärteilchendurchmessers ist, der mit einem Transmissionselektronenmikroskop gemessen wird, und

ein Elutionsverhältnis S des Zinkoxids, das durch den folgenden Ausdruck (1) definiert wird, 50% oder weniger beträgt, wenn in einer Pufferlösung mit einem pH-Wert von 5 für 1 Stunde gehalten:

```
Elutionsverhältnis S = (Menge an eluiertem
Zinkoxid)/(Menge an Zinkoxid im Siliziumoxid-beschichtetem
Zinkoxid) (1).
```

**2.** Das mit Siliziumoxid beschichtete Zinkoxid gemäß Anspruch 1,
wobei der durchschnittliche Primärteilchendurchmesser der Zinkoxidteilchen 200 nm oder mehr und 500 nm oder weniger beträgt.

**3.** Das mit Siliziumoxid beschichtete Zinkoxid gemäß Anspruch 1 oder 2,
wobei die Siliziumoxidbeschichtung mindestens ein Element ausgewählt aus der Gruppe bestehend aus Magnesiumsalzen, Kalziumsalzen und Bariumsalzen umfasst.

**4.** Das mit Siliziumoxid beschichtete Zinkoxid gemäß einem der Ansprüche 1 bis 3,
wobei der durchschnittliche Primärteilchendurchmesser der Zinkoxidteilchen 200 nm oder mehr und 300 nm oder weniger beträgt, und
90% oder mehr der Zinkoxidteilchen Primärteilchendurchmesser von 100 nm oder mehr und 400 nm oder weniger aufweisen.

**5.** Ein Verfahren zur Herstellung von mit Siliziumoxid beschichtetem Zinkoxid, umfassend:

Mischen einer wässrigen Zinkoxidsuspension, die Zinkoxidteilchen mit einem durchschnittlichen Primärteilchendurchmesser von mehr als 50 nm und 500 nm oder weniger enthält, mit einer wässrigen Silikatalkalimetallsalzlösung, die ein Silikatalkalimetallsalz enthält, um eine wässrige Suspension herzustellen, die die Zinkoxidteilchen und das Silikatalkalimetallsalz enthält, und Zugabe einer Säure zur wässrigen Suspension, um eine Reaktion zu bewirken;
und
thermische Behandlung des erhaltenen Reaktionsprodukts bei einer Temperatur von 200°C oder höher und niedriger als 600°C für eine bis fünf Stunden.

**6.** Verfahren zur Herstellung von mit Siliziumoxid beschichtetem Zinkoxid gemäß Anspruch 5,
wobei eine wässrige Lösung, die mindestens eines ausgewählt aus der Gruppe bestehend aus Magnesiumsalzen, Kalziumsalzen und Bariumsalzen umfasst, zu einer Reaktionslösung, die durch Zugabe der Säure erhalten wurde, zugegeben und mit ihr gerührt wird und die Mischung ein bis fünf Stunden stehen gelassen wird, wodurch eine Substitutionsreaktion durchgeführt wird.

**7.** Eine Zusammensetzung, die mit Siliziumoxid beschichtetes Zinkoxid enthält, umfassend:
das mit Siliziumoxid beschichtete Zinkoxid gemäß einem der Ansprüche 1 bis 4.

**8.** Ein kosmetisches Material, umfassend:
mindestens eines der mit Siliziumoxid beschichteten Zinkoxide gemäß einem der Ansprüche 1 bis 4 und die Zusammensetzung gemäß Anspruch 7, die mit Siliziumoxid beschichtetes Zinkoxid enthält, in einer Basis.

**Revendications**

**1.** Oxyde de zinc recouvert d'oxyde de silicium formé en recouvrant des surfaces de particules d'oxyde de zinc avec un revêtement d'oxyde de silicium,
dans lequel la quantité d'oxyde de silicium dans l'oxyde de zinc recouvert d'oxyde de silicium est de 12 % en masse ou plus,

un diamètre moyen de particules primaires des particules d'oxyde de zinc est supérieur à 50 nm et 500 nm ou moins, le diamètre moyen de particules primaires des particules d'oxyde de zinc est une valeur moyenne en masse d'un diamètre de particules primaires mesuré en utilisant un microscope électronique à transmission, et

en cas de maintien dans une solution tampon ayant un pH de 5 pendant 1 heure, un taux d'élution S de l'oxyde de zinc défini par l'expression (1) suivante est de 50 % ou moins :

$$\text{taux d'élution } S = (\text{quantité d'oxyde de zinc élué}) / (\text{quantité d'oxyde de zinc dans}$$

$$\text{un oxyde de zinc recouvert d'oxyde de silicium}) \ (1).$$

2. Oxyde de zinc recouvert d'oxyde de silicium selon la revendication 1, dans lequel le diamètre moyen de particules primaires des particules d'oxyde de zinc est de 200 nm ou plus et de 500 nm ou moins.

3. Oxyde de zinc recouvert d'oxyde de silicium selon la revendication 1 ou 2, dans lequel le revêtement d'oxyde de silicium comprend au moins un élément sélectionné à partir du groupe constitué par des sels de magnésium, des sels de calcium, et des sels de baryum.

4. Oxyde de zinc recouvert d'oxyde de silicium selon l'une quelconque des revendications 1 à 3, dans lequel le diamètre moyen de particules primaires des particules d'oxyde de zinc est de 200 nm ou plus et de 300 nm ou moins, et des diamètres de particules primaires de 90% ou plus des particules d'oxyde de zinc sont de 100 nm ou plus et de 400 nm ou moins.

5. Procédé de préparation d'oxyde de zinc recouvert d'oxyde de silicium, comprenant:

un mélange d'une suspension à base d'eau d'oxyde de zinc incluant des particules d'oxyde de zinc ayant un diamètre moyen de particules primaires supérieur à 50 nm et 500nm ou moins avec une solution aqueuse de sel de métal alcalin de silicate incluant un sel de métal alcalin de silicate de façon à préparer une suspension à base d'eau incluant les particules d'oxyde de zinc et le sel de métal alcalin de silicate, et un ajout d'un acide à la suspension à base d'eau pour entraîner une réaction ; et

un traitement thermique du produit de réaction obtenu à une température de 200 °C ou plus et inférieure à 600 °C pendant une à cinq heures.

6. Procédé de préparation d'oxyde de zinc recouvert d'oxyde de silicium selon la revendication 5, dans lequel une solution aqueuse incluant au moins un élément sélectionné à partir du groupe constitué par des sels de magnésium, des sels de calcium et des sels de baryum est ajoutée et remuée avec une solution de réaction qui a été obtenue en ajoutant l'acide, et le mélange décante pendant une à cinq heures, effectuant ainsi une réaction de substitution.

7. Composition contenant de l'oxyde de zinc recouvert d'oxyde de silicium, comprenant : l'oxyde de zinc recouvert d'oxyde de silicium selon l'une quelconque des revendications 1 à 4.

8. Produit cosmétique comprenant : au moins un parmi l'oxyde de zinc recouvert d'oxyde de silicium selon l'une quelconque des revendications 1 à 4 et la composition contenant de l'oxyde de zinc recouvert d'oxyde de silicium selon la revendication 7 dans une base.

FIG. 1

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0824086 A1
- JP 2851885 B
- JP 3848458 B
- JP 3520785 B
- JP 4836232 B
- WO 2014171322 A